# EUROPEAN PATENT APPLICATION

(11) **EP 3 366 310 A1**
(43) Date of publication of application: **29.08.2018**
(21) Application number: 17305197.0
(22) Date of filing: 23.02.2017
(51) Int. Cl.: A61K 45/06, A61K 31/7004, A61P 31/04

(54) **L-ARABINOSE AND USES THEREOF AGAINST VIBRIO GENUS BACTERIA**

(71) Applicant: Centre National de la Recherche Scientifique, 75016 Paris (FR); Universite Paris-Sud, 91400 Orsay (FR)
(72) Inventor: BARRE, François-Xavier, 91190 GIF SUR YVETTE (FR); GALLI, Elisa, 91400 ORSAY (FR); ESPINOSA ALFARO, Elena, 92340 BOURG LA REINE (FR)
(74) Representative: Regimbeau

(57) **Abstract**

The invention relates to the field of antibacterial compositions. The invention more particularly relates to bacteriostatic or bactericidal products as well as to compositions comprising such products. It further relates to a bacteriostatic or bactericidal treatment of a subject or medium and to a method for assessing the sensitivity of a bacterial strain to a bacteriostatic or bactericidal product and more particularly to L-Arabinose or a derivative thereof.

## Description

The invention relates to the field of antibacterial compositions. The invention more particularly relates to bacteriostatic or bactericidal products as well as to compositions comprising such products. It further relates to the bacteriostatic or bactericidal treatment of a subject or medium, and to a method for assessing the sensitivity of a bacterial strain to a bacteriostatic or bactericidal product as herein described, in particular L-Arabinose or a derivative thereof.

### BACKGROUND

*Vibrio* genus bacteria are ubiquitous in the aquatic environment and are typically present in water, in particular seawater, as well as in shellfish/crustaceans, seafood, etc. Some of them are able to cause human diseases, including the cholera toxin-producing strains of *Vibrio cholerae* that are responsible for cholera. Cholera occurs in both endemic and epidemic pattern. A cholera-endemic area is an area where confirmed cholera cases were detected during three out of the last five years with evidence of local transmission. A cholera outbreak/epidemic is defined by the occurrence of at least one confirmed case of cholera with evidence of local transmission in an area where there is not usually cholera. In recent years, devastating epidemics of cholera have occurred in Angola, Ethiopia, Zimbabwe, Pakistan, Somalia, Sudan, Vietnam, and Haiti. Cholera transmission is closely linked to inadequate access to clean water and sanitation facilities. Cholera affects an estimated 1.3-4 million people worldwide and causes 21,000-143,000 death a year according to the World Health Organization. While the risk of death is usually less than 5%, it may be as high as 50% among some groups who do not have access to treatment. Cholera can kill a subject, in particular a human being, within hours if left untreated.
Cholera outbreaks can be initiated by transmission of pathogenic strains of *V. cholerae* from their environmental reservoir to humans through seafood or other environmental related food or water sources.
After ingestion of *V. cholerae*, the majority of bacterial germs are killed by gastric acid. Surviving organisms colonize the small intestine and elaborate cholera toxin, the major virulence factor for pathogenic strains of *V. cholerae.* The symptoms are profuse diarrhea and vomiting of clear fluid and they usually start half a day to five days after ingestion of the bacteria. An untreated person may produce 10 to 20 liters of diarrhea a day, which may result in life-threatening dehydration and electrolyte imbalances. Severe cases therefore need rapid treatment with intravenous fluids and antibiotics. Antibiotics, such as doxycycline, cotrimoxazole or erythromycin, are routinely used to diminish the duration of diarrhea, reduce the volume of rehydration fluids needed, and shorten the amount and duration of *V. cholerae* excretion in the patient's stools. However, mass administration of those antibiotics is not recommended by WHO as it contributes to increase antimicrobial resistance.
In addition to humans, *Vibrio* species are also pathogenic for many fish, shellfish or crustacean species. For example, *V. aestuarianus*, *V. alginolyticus* and *V. splendidus* are responsible for oysters' illness. Typically, *V. alginolyticus* leads to tissue necrosis and increases oyster death rate in culture. This represents an important economic loss for farmers. However, the use of antibiotics in oyster or other mussels' cultures involves a risk of spreading antibiotic-resistance genes in the environment.
Due to the high infectivity and the possibly lethal consequences of *Vibrio* species, there is a need for new products, in particular therapeutic products, preferably prophylactic products, usable in a subject or medium who/which has been, or could be, in contact with, or exposed to, an infected or possibly infected distinct person or medium. The use of a prophylactic product is particularly advantageous in that it restrains the propagation of bacteria. However, such a prophylactic product should be very specific so that it does not lead to major side effects, typically to the appearance of resistances in non-targeted bacterial strains, of intestinal disorders (such as, e.g., diarrhea, nausea, or vomiting), or of mycosis (such as, e.g., candidiasis).

### BRIEF DESCRIPTION OF THE INVENTION

Arabinose is an aldopentose. It consists of a monosaccharide containing five carbon atoms and including an aldehyde functional group. As most saccharides, two enantiomeric forms coexist, a D-form ("D-Ara") and an L-form ("L-Ara"). L-arabinose is more common than D-arabinose in nature. It is widely distributed in terrestrial plants as a pectin sugar and as a component of the hemicellulose of both the primary and secondary cell walls of plants, including woods and cereal grains. Many bacteria encode for an L-arabinose catabolic pathway. In addition, enzymes from the *Escherichia coli* L-arabinose catabolic pathway have permitted to develop a tight inducible expression system, P_{BAD}, which is utilized in a wide variety of bacterial genus, including Enterobacteriaceae and Vibrionaceae. Inventors herein reveal that concentrations of L-Arabinose (L-Ara) as low as 0.02% trigger a toxic response in *Vibrio cholerae (V. cholerae*): cells stop to proliferate, lose their curved rod-shape and transition to cell wall deficient spheres. The cell wall deficient spheres are similar in appearance and in residual peptidoglycan composition to L-form bacteria. Part of these spheres can revert to a wild-type morphology once L-Ara is removed from the growth medium and re-start proliferation. However, L-Ara treatment leads to about 50% of cell death.

Inventors herein describe for the first time the use of L-Ara or of a (functional) derivative thereof as an active bacteriostatic or bactericidal compound.

Objects herein described thus relate to a bacteriostatic or bactericidal product consisting in, or comprising, at least one active compound consisting in L-Ara or a derivative thereof, to compositions and kits comprising such a bacteriostatic or bactericidal product, as well as to uses thereof, typically for preventing, treating or alleviating a bacterial infection in a subject in need thereof. The compositions and kits preferably comprise L-Ara or a derivative thereof together with a pharmaceutically acceptable excipient, carrier or support. Those products are particularly useful against bacterial strains that do not express L-Ara catabolic enzymes, such as *Vibrio* genus bacteria. A typical example of such bacterial strain is *Vibrio cholerae.* Another typical example of such bacterial strain is *Vibrio parahaemolyticus.*

Also herein described is a process for the bacteriostatic or bactericidal *in vivo*, *in vitro* or *ex vivo* treatment of a medium infected with, or at risk of being infected with, a bacterium or bacteria, wherein the process comprises a step of contacting the infected medium with a bacteriostatic or bactericidal product or with a composition as herein described.

Inventors in addition herein describe a method for assessing *in vitro* or *ex vivo* the sensitivity of a bacterial strain to L-Ara or to a derivative thereof, wherein the method comprises a step of exposing the bacterial strain to L-Ara or to a derivative thereof, and a step of assessing the shape, peptidoglycan content and/or proliferation rate of the bacteria, thereby assessing the sensitivity of the bacterial strain to L-Ara or to a derivative thereof.

Further herein described is a kit comprising at least one bacteriostatic or bactericidal product or a composition as herein disclosed preferably together with a product and/or tool adapted to the detection and/or measure of L-Ara or of a derivative thereof in a medium or in a biological sample of a subject, and/or preferably together with at least one glycoside hydrolase.

Such a kit can advantageously be used for the bacteriostatic or bactericidal treatment of a subject or medium, and/or for detecting and/or measuring L-Ara and/or L-Ara derivatives levels in a biological sample of the subject or in the medium or a sample thereof.

### DETAILED DESCRIPTION OF THE INVENTION

The use of inducible promoters in bacterial studies has become a very useful tool to investigate the expression or depletion of genes. A wide array of promoters has been devised to modulate the expression levels and to suit different bacterial hosts as it is often desirable to coordinate the concurrent expression of multiple genes at once. Widely used expression systems are based on the inducible promoters P_{LAC} and P_{BAD}. The P_{BAD} system is especially advantageous for its tightly regulated expression [Guzman *et al.*]. It is based on the *Escherichia coli* promoter of the *araBAD* operon (from which it derives its name) and the gene *araC*, encoding the protein that regulates the *araBAD* promoter [Lee *et al.*]*.* The AraBAD enzymes are necessary for the arabinose catabolism and are expressed only in presence of their substrate [Lee *et al.*]*.* AraC acts both as a positive and a negative regulator, repressing the P_{BAD} promoter in the absence of the inducer and activating its transcription upon binding to L-Ara [Lee *et al.*]*.* Expression of the P_{BAD} promoter is also regulated by the presence of glucose, which can decrease the cyclic-AMP levels until the leakiness of the promoter is repressed [Miyada *et al.*]*.*
Inventors studied the processes of DNA synthesis, chromosome segregation and cell division in *Vibrio cholerae*, the agent of the deadly diarrheal human disease cholera. To this end, they integrated *araC* and the *araBAD* promoter in the genome of *V. cholerae* strains to investigate the ectopic expression of recombinant proteins, a common practice in research on the *Vibrio* genus. No deleterious effect was ever reported on *V. cholerae.* On the contrary, experiments on the squid symbiont *Vibrio fischeri* suggested that L-Ara could up-regulate a protective response against antimicrobial agents, such as those produced by seaweed, via the induction of biofilm formation [Visick *et al.*]*.* Inventors were therefore very surprised to observe that the addition of L-Ara inhibited the growth and generated morphological defects in several V. *cholerae* mutants. Inventors confirmed that the observed phenotype was not due to the particular genotype of the mutants nor to the integration of *araC* and the Ara promoter in the bacterial genome, as wild type cells were susceptible to the same morphological changes in the presence of L-Ara, even though it was necessary to increase the L-Ara concentration in order to observe a similar detrimental response.

Inventors thus herein describe for the first time a bacteriostatic or bactericidal product comprising at least one active compound consisting in L-arabinose (L-Ara) or a derivative thereof, typically a natural or artificial derivative thereof. In a particular aspect, the bacteriostatic or bactericidal product of the invention consists in L-Ara or a natural or artificial derivative thereof.
A bacteriostatic product is a product that stops bacteria from reproducing, while not necessarily killing them. Such a product may be used as antibiotic, disinfectant, antiseptic or preservative. When used as antibiotic, the treatment must be long enough to allow host defense mechanisms to eradicate the bacteria. A bacteriostatic antibiotic typically limits the growth of bacteria by interfering with bacterial protein production or DNA replication. Upon removal of a bacteriostatic product, bacteria usually start to grow again. In contrast, a bactericide is a substance that kills bacteria and which is commonly used as a disinfectant, antiseptic or antibiotic.
Inventors herein demonstrate for the first time that L-Ara is toxic for *Vibrio* genus bacteria and induces the formation of spherical cells that are no longer able to proliferate. The spherical forms of said bacteria are similar to L-form bacteria. In particular, they have almost entirely lost (spheroplast-type) their peptidoglycan layer. L-forms phenotype can develop from Gram-positive and from Gram-negative bacteria upon action of an antibiotic such as penicillin.
In the context of the invention the term "active compound" designates a compound that has a bacteriostatic and/or bactericidal activity. A compound has a bacteriostatic activity against bacteria when said bacteria are no longer able to proliferate upon exposure to said compound. A compound has a bactericidal activity when it induces death of at least 40%, 50%, 60%, 70%, 80%, 90%, 95%, 96%, 97%, 98%, or 99% of the bacteria present in the infected subject or medium.
The term "L-Ara derivative" designates active compounds resulting from the catabolic processing of L-Ara by a cell (L-Ara by-products). Such derivatives typically result from the degradation of L-Ara all along the arabinose catabolic pathway, the galactose catabolic pathway or the glycolysis pathway. Typical naturally occurring L-Ara derivatives are for example L-ribulose, L-ribulose 5-phosphate and D-xylulose 5-phosphate. L-Ara derivatives also refer to L-Ara and by-products thereof which have been modified, typically artificially modified, for example chemically modified, by the addition or deletion of functional or non-functional groups without interfering with/affecting the bacteriostatic or bactericidal effect of the active compound. Preferred examples of artificial modifications include among others methylation, esterification, acetalisation, acylation, aldol-addition, alkylation, alkynylation, sulfonation, sulfonylation, sulfinylation, nitration, nitrilation, nitrosation, hydrohalogenation, arylation, carbonylation, carboxylation, hydroxylation, amination or amidation of a L-Ara derivative, or addition of iminomethoxy groups, phosphate groups, phosphate ester groups, aminothiazole groups, or halo-, fluoro-, chloro-, bromo-, iodo- groups to a L-Ara derivative.

In a particular aspect, the bacteriostatic or bactericidal product herein described is, or can be used as, a medicament, a disinfectant, an antiseptic or a preservative agent.
In a preferred aspect, the medicament is an antibiotic. Such a medicament kills (bactericide product) or at least limits the growth (bacteriostatic product) of bacteria, and can be used in the prevention or treatment of a bacterial infection.
A disinfectant is an antimicrobial agent that is applied to the surface of non-living objects (also herein identified as "medium") to destroy microorganisms that are living on the objects/media.
An antiseptic is an antimicrobial substance that is applied to living tissue/skin to reduce the possibility of infection, sepsis, or putrefaction. Antiseptics are generally distinguished from antibiotics by the latter's ability to be transported through the lymphatic system to destroy bacteria within the body, and from disinfectants, which destroy microorganisms found on non-living objects.
In the context of the invention, a preservative is a substance that is added to products such as food, beverages, pharmaceutical drugs, paints, biological samples, cosmetics, wood, and many other products to prevent decomposition by microbial growth. Antimicrobial preservatives prevent products' degradation by bacteria.

Inventors also herein describe the use of a product as described herein above, typically L-Ara or a herein described derivative thereof, to prepare a composition, typically a medicament, a disinfectant, an antiseptic or a preservative agent.
A particular object herein described is thus a composition comprising a product as described herein above, typically L-Ara or a derivative thereof, in a bacteriostatic or bactericidal effective amount, and an excipient, carrier or support, typically a pharmaceutically acceptable excipient, carrier or support. Such a composition, may further advantageously comprise an enzyme damaging the bacterial cell wall and/or capsule, typically a glycoside hydrolase such as a lysozyme, a cellulase, a lipase, preferably a lysozyme (also herein identified as muramidase).
Typically, a bacteriostatic or bactericidal effective amount refers to the quantity of product necessary for obtaining the bacteriostatic or bactericidal effect.
In a preferred aspect, the amount of L-Ara or of a derivative thereof present in the herein described composition is between 0.1% and 50%, and is even more preferably of 10% when the composition is to be used as a medicament or as a disinfectant. This amount is typically between 0.1% and 50%, preferably of 10%, when the composition is to be used as an antiseptic or as a preservative agent. These amounts can be easily adapted by the skilled person of the art depending on the subject or medium which is to be exposed to the claimed product.
Compositions according to the present description may typically include any diluent, excipient, carrier or support. Compositions may for example include thickeners, buffers, surface active agents and the like. Such products do not abrogate the biological activity of the herein described bacteriostatic or bactericidal products and preferably consist in pharmaceutically acceptable solutions or vehicles, i.e. physiologically acceptable solutions or vehicles that are harmless or do not cause any significant specific or non-specific immune reaction to living organisms. For example, liquid formulation, saline water, sterile water, Ringer's solution, buffered physiological saline solution, albumin infusion solution, dextrose solution, maltodextrin solution, glycerol, ethanol, and mixtures thereof may be used as a pharmaceutically acceptable diluent, excipient, carrier or support.

When used as a medicament, the composition is preferably formulated as an oral, topical or injectable composition.
Diluents, dispersants, surfactants, binders and lubricants may be additionally added to the composition to prepare oral formulations such as pills, capsules, granules, or tablets, or powdered formulations, or to prepare injectable formulations such as aqueous solutions, suspensions, and emulsions. Formulations for topical administration may include, band aids, dressings, patches, films, ointments, lotions, creams, gels, drops, suppositories, sprays, aerosols, tampons, sanitary towels, liquids and powders.
When used as an antiseptic, the composition is preferably formulated as a topical composition. Typically, the composition is formulated as a gel, a cream, a spray or an aerosol. When used as a disinfectant, the composition is preferably formulated as a liquid, a gel, a lotion, a spray or an aerosol.
When used as an antimicrobial preservative, the composition is preferably formulated as a liquid, a gel, a lotion, a spray or an aerosol.

In a particular aspect, the herein described bacteriostatic or bactericidal product or the composition comprising such a product is for use for preventing or treating a bacterial infection in a subject, or for preparing a composition for preventing or treating a bacterial infection in a subject.
The terms "preventing or treating" designate a curative or a prophylactic treatment of a bacterial infection. A curative treatment is defined as a treatment that results in the cure of a bacterial infection, or a treatment that alleviates, reduces, stabilizes, or eliminates the symptoms of an infectious disease or the suffering that it causes, directly or indirectly, or that improves a subject condition or reduces progression of a bacterial infection. A prophylactic treatment designates a treatment resulting in the prevention of a bacterial infection, and/or a treatment reducing and/or delaying the incidence of a bacterial infection or the risk of its occurrence. The prevention of infection may for example result from the bacteriostatic effect of the described compound or composition, which inhibits the growth of bacteria, said bacteria thus being unable to infect a subject and provoke measurable symptoms or unable to proliferate in a medium. This prophylactic effect is for example very useful for preventing subjects who have been in contact with an infected or possibly infected subject from developing an infection.
In the context of the invention, the "subject" is an animal. Preferably the animal is a fish, a shellfish, a crustacean or a mammal.
Examples of fishes include eels, salmonids, turbots, goldfishes, mackerels, cods, sturgeons, sea-basses, or guilt-head sea-breams.
Shellfish designates aquatic shelled mollusks, typically edible shelled mollusks, and include for example oysters, cockles, mussels, sea snails, St James shells, or chitons.
Examples of crustaceans include crabs, shrimps, lobsters, and crayfishes.
Examples of mammals include humans and non-human animals such as, without limitation, domesticated animals (e.g., cows, sheep, pigs, rabbits, cats, dogs, and horses), non-human primates (such as monkeys), and rodents (e.g., mice and rats).
In a particular embodiment, the subject is a human being.

In another particular embodiment, the subject is an edible shellfish or crustacean, preferably an oyster.

In a typical aspect, a bacterium targeted by the herein described bacteriostatic or bactericidal products and/or compositions is a bacterial strain that does not express L-Ara catabolic enzyme(s) such as for example L-arabinose isomerase (AraA), L-ribulokinase (AraB) and L-ribulose 5-phosphate 4-epimerase (AraD).
As demonstrated hereinafter by inventors, once L-Ara or a derivative thereof enters the bacteria, it is catabolized through the galactose catabolic and/or glycolysis pathways. Processing results in the apparition of L-Ara bacteria-toxics by-products.
Inventors herein describe a method for assessing *in vitro* or *ex vivo* the sensitivity of a bacterial strain to L-Ara or to a derivative thereof.
In the context of the present invention, bacteria are typically selected from the *Vibrio* genus. Preferred *Vibrio* bacteria are for example selected from *Vibrio cholerae*, *V. parahaemolyticus*, *V. vulnificus,* which are human pathogens, i.e. which may cause a disease or disorder in a human being, and from any *Vibrio* species causing a disease or disorder in a fish and/or shellfish, such as *V. aestuarianus, V. alginolyticus*, *V. splendidus* and *V. harveyi*, which are common causes of mortality among domestic marine life.
In a preferred aspect of the invention, the targeted bacterium or bacteria is (are) selected from *Vibrio cholerae*, *V. parahaemolyticus*, *V. vulnificus, V. aestuarianus, V. alginolyticus*, *V. splendidus*, *V. Harveyi* and any combination thereof, such as a combination of *Vibrio cholera*, *V. parahaemolyticus, V. vulnificus* or a combination of *V. aestuarianus, V. alginolyticus*, *V. splendidus* and *V. harveyi.*

One of the advantages offered by the herein described active products and compositions resides in that L-Ara is a well-known compound which does not affect most of the bacterial genus, in particular most of the commensal bacterial flora. Thus, the equilibrium of the bacterial flora is not affected when a subject is exposed to or treated with L-Ara. This avoids appearance of side effects such as the development of mycosis, for example candidiasis, or of intestinal disorders such as diarrhea, nausea or vomiting in the exposed/treated subject. Moreover, since most of the bacteria are not sensitive to L-Ara, it considerably reduces the risk of development of bacterial resistance in bacteria distinct from the targeted bacterial strain.

Also herein described is a method or process for the bacteriostatic or bactericidal treatment of a subject (as herein described) or of a medium infected with, or at risk of being infected with, a bacterium as described herein above, wherein the process comprises a step of contacting the subject or medium with a bacteriostatic or bactericidal product or with a composition comprising such a product as herein disclosed.

The terms "medium" and "media" herein refer to a solid, semi-solid or liquid medium/media. A typical example of medium that can be infected by bacteria as herein described is water such as seawater, fresh water, tap water, a culture cell water or an animal culture water. In another typical example, the medium is a culture cell medium or an aquatic animal culture medium, in particular a seafood culturing water, for example oysters culturing water.
In a preferred embodiment, the medium is selected from sea-water, fresh water, tap water, culture cell water or medium, and an aquatic animal culture water or medium.
Use of L-Ara in said cultures allows via a prophylactic and/or curative effect to grow healthier oysters and to avoid the transmission of pathogenic bacteria, for example the transmission of *V. cholerae*, between oysters and to the subject who will eat them, typically the human consumer.
Another particular example of medium is a physiological medium, typically a subject's biological sample such as for example a blood, lymph, skin, mucous, or intestinal bowel sample, for example a sample of stools or excretions.

Also herein disclosed is a method for assessing or evaluating *in vitro* or *ex vivo* the sensitivity of a bacterial strain to L-Ara or to a derivative thereof, wherein the method comprises a step of exposing the bacterial strain to L-Ara or to a derivative thereof, and a step of studying the bacterial cells, typically of assessing the bacterial cell shape, peptidoglycan (also herein identified as "muropeptide" or "murein") content and/or proliferation rate, thereby assessing the sensitivity of the bacterial strain to L-Ara or to a derivative thereof. The bacterial cell wall integrity, structure or composition may also be evaluated to assess the sensitivity of the bacterial strain to L-Ara or to a derivative thereof.

Typically, the conversion of bacteria into protoplasts or spheroplasts, a peptidoglycan content of bacteria, typically of spheroplasts, below 20%, in particular below 10%, and/or the bacterial cell wall degradation and/or remodeling, indicates the sensitivity of the bacterial strain to L-Ara or to a derivative thereof. On the contrary, the absence of any change in the bacteria's shape and/or cell wall, or a peptidoglycan content of the bacteria above 10%, in particular above 20%, indicates the resistance of the bacterial strain to L-Ara or to a derivative thereof.

The bacteria's shape as well as cell wall degradation and/or remodeling may be assessed by any methods known by the skilled person. Typically, snapshot analysis acquired using a microscope and/or time-lapse analysis acquired using a camera may be used. Alternatively, resistance to osmotic stresses can be monitored by subjecting bacterial cells to an osmotic downshift (Joseleau-Petit et al. 2007).

The peptidoglycan (also herein identified as muropeptide or murein) content of the bacteria strain may be assessed using any methods known by the skilled person.
Peptidoglycan is a polymer consisting of sugars and amino acids that forms a mesh-like layer outside the plasma membrane of most bacteria, forming the cell wall. The sugar component consists of alternating residues of β-(1,4) linked N-acetylglucosamine and N-acetylmuramic acid. Peptidoglycan serves a structural role in the bacterial cell wall, giving structural strength, as well as counteracting the osmotic pressure of the cytoplasm. The peptidoglycan layer is substantially thicker in Gram-positive bacteria (20 to 80 nanometers) than in Gram-negative bacteria (7 to 8 nanometers). Peptidoglycan is also important in cell fission during bacterial cell reproduction. Reduction of peptidoglycan production is involved in the action of some antibacterial drug such as penicillin.
Peptidoglycan content may be analyzed using ultra performance liquid chromatography (UPLC) and mass spectrometry analysis as described in Desmarais *et al.*, Möll *et al.*, and Glauner *et al.* Alternatively, the peptidoglycan content of the bacteria may be assessed using for example a radiochemical molecule, typically a radiomarked peptidoglycan precursor the incorporation of which in the bacterial cell wall may be observed and measured (cf. Wientjes *et al*.).

Bacterial proliferation rate may be measured using any methods known by the skilled person. Typical methods for measuring bacterial proliferation rate comprise counting methods and methods based on the determination of the bacterial biomass.
Counting methods may be carried out by direct counting using a microscope at t0 and t1 and typically include a step of comparing the said measurements at t0 and t1.

In another aspect, the counting may be carried out with colony-forming unit (CFU). Counting with colony-forming units requires culturing the microbes and counting only viable cells, in contrast with microscopic examination which counts all cells, living or dead. In such an assay, CFU is measured at t0 and t1 and the ratio of CFU at t1/CFU at t0 indicates the proliferation rate.
Typically, a bacterial proliferation rate below 60%, typically below 50%, indicates the sensitivity of the bacterial strain to L-Ara or to a derivative thereof, and a bacterial proliferation rate above 60%, typically above 65%, 70% or 75%, preferably above 80%, indicates the resistance of the bacterial strain to L-Ara or to a derivative thereof.

An additional object of the present disclosure is a kit comprising at least one bacteriostatic or bactericidal product or composition as disclosed herein above, for example two bacteriostatic or bactericidal products or compositions as disclosed herein above, preferably together with at least one glycoside hydrolase as herein described, and preferably together with a product and/or tool adapted to the detection and/or measure of L-Ara or of a derivative thereof in a biological sample of a subject or in a medium or sample thereof.
The glycoside hydrolase can for example be selected from a lysozyme, a cellulase and a lipase. The additional product can for example be selected from any compound damaging or degrading the bacterial cell wall and/or capsule of Gram-positive and/or Gram-negative bacteria.
The tool can for example be selected from a solution comprising a biological revelator or a chemical revelator of the presence of L-Ara or a derivative thereof, or a combination thereof, said revelator(s) being each aliquoted in ready-to-use containers or deposited or impregnated on solid support(s). Such a tool can be advantageously used to detect or measure the concentration of L-Ara or of any derivative thereof in a biological sample of a subject or in a medium or sample thereof.

Also herein disclosed is the use of such a kit for the bacteriostatic or bactericidal treatment of a subject or medium, typically of a subject or medium as herein defined.
Such a kit can also advantageously be used for detecting and/or measuring L-Ara levels or L-Ara derivatives levels in the medium or in a subject's biological sample.
In a preferred aspect, the subject is a human being.
In another aspect, the medium is selected from sea water, fresh water, tap water, a culture cell water or medium and an aquatic animal culture water or medium.

The examples, which follow, and their corresponding figures illustrate the invention without limiting the scope thereof.

### FIGURES

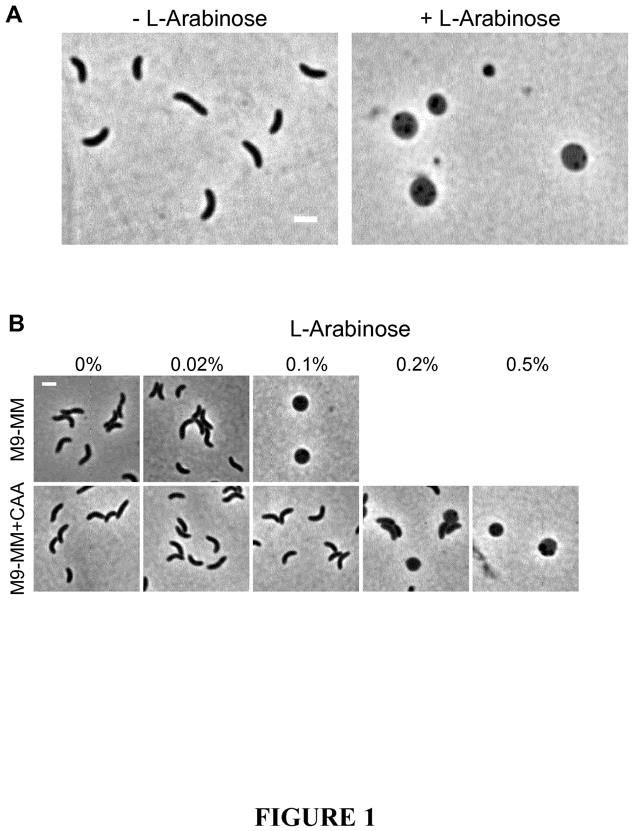

**Figure 1****. L-Ara induces loss of rod-shape.**
   **A.** *V. cholerae* N16961 cells were grown in M9-MM. In the absence of L-Ara, cells are rod-shaped (left panel), in the presence of 0.1% L-Ara they have a spherical shape with a heterogeneous diameter size (right panel). **B.** *V. cholerae* N16961 cells were grown in M9-MM, M9-MM supplemented with CAA with increasing concentrations of L-Ara. Cells were inspected for spherical morphology. Error bars = 2 µm.
**Figure 2****. L-Ara induces inhibition of cell growth.**
   *V. cholerae* N16961 growth profiles in M9-MM (top panel) and M9-MM + CAA (bottom panel) in the presence of increasing concentrations of L-Ara. The optical density at 600 nm over time is the mean of three independent replicates; the standard deviation is represented for each time point.
**Figure 3****. Formation of cell wall-deficient (CWD)-cells and recovery of rod shape.**
   Still images from time-lapse microscopy experiments. Images were taken every 5 minutes for 14 hours. **A.** Transition from rods to CWD-forms. N16961 cells were grown in M9-MM to exponential phase and mounted on an M9-MM agarose pad containing 0.2% L-Ara. **B.** Recovery from CWD-forms to rods. N16961 cells were grown in M9-MM + 0.2% L-Ara until they became spherical and then mounted on a M9-MM agarose pad in the absence of L-Ara. Error bars = 2 µm.

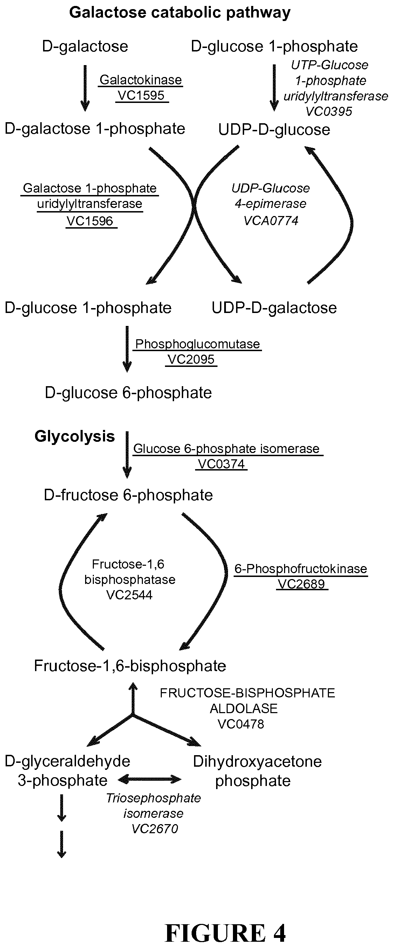

**Figure 4****. L-Arabinose toxic by-product is produced through the galactose and glycolysis metabolic pathways.**
   Schematic representation of the putative galactose Leloir and glycolysis metabolic pathways in *V. cholerae.* Underlined are the genes that when inactivated suppress the L-Ara induced phenotype, in italics the non-suppressors and in capital letters the gene that could not be inactivated because essential.

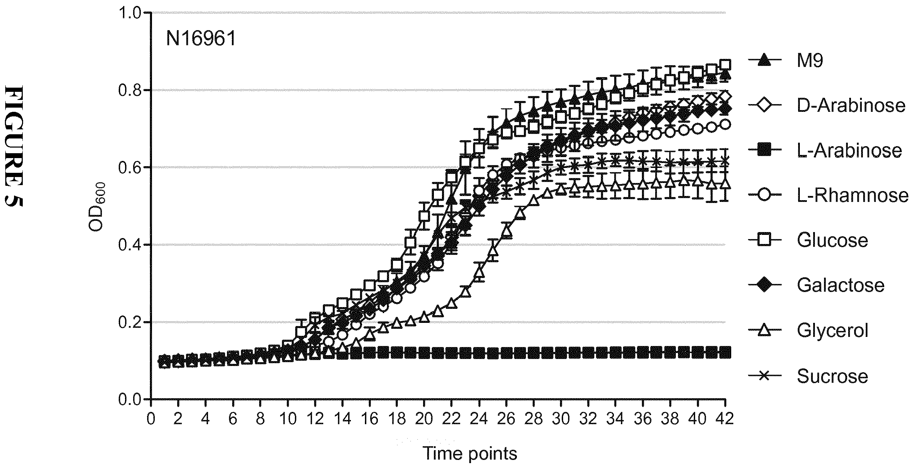

**Figure 5****. Carbon sources tested for inhibition of cell growth.**
   *V. cholerae* N16961 growth profiles in M9-MM in the presence of 0.2% of different carbon sources. The optical density at 600 nm over time is the mean of three independent replicates; the standard deviation is represented for each time point.

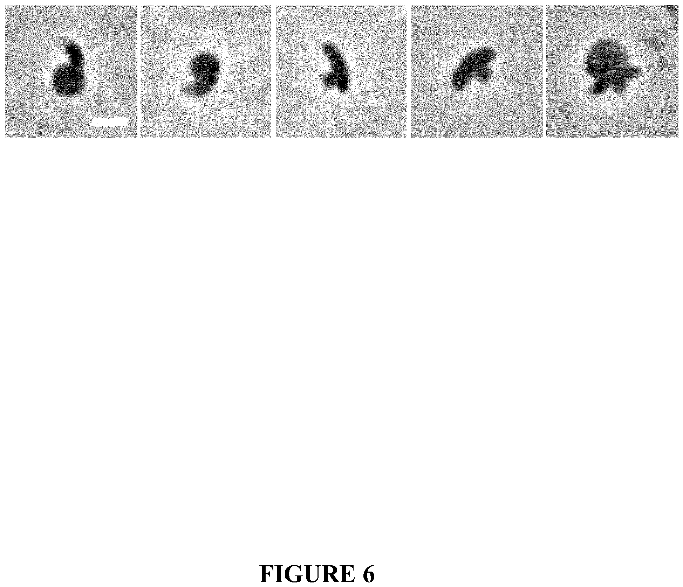

**Figure 6****. Localization of blebs after N16961 exposure to 0.2% L-Ara.**
   Snapshots images of *V. cholerae* cells transitioning to CWD-forms after exposure to 0.2% L-Ara. Error bar = 2 µm.

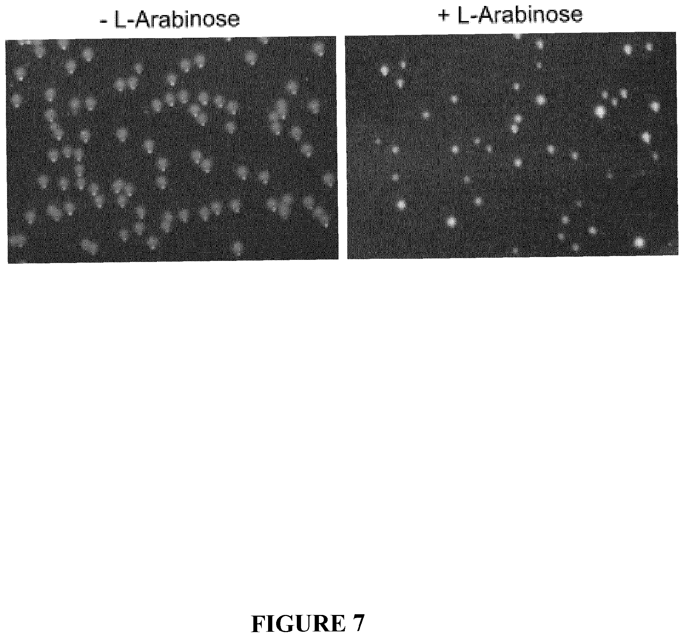

**Figure 7****. Colony morphology.**
   Colony morphology after liquid treatment with 0.2% L-Ara (right panel) and without treatment (left panel). Samples were plated on LB plates in the absence of L-Ara and pictures of plates acquired.

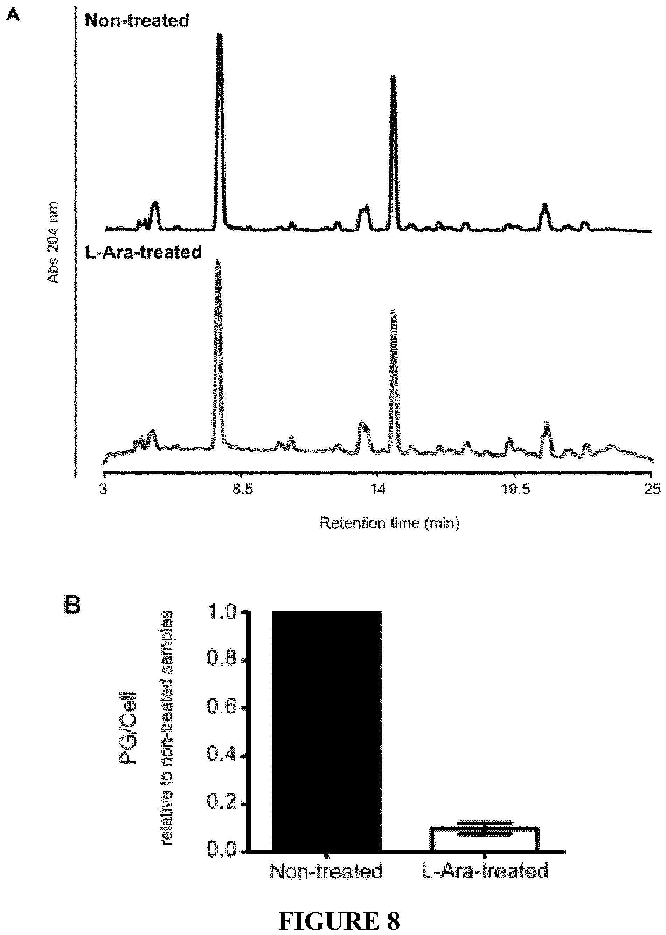

**Figure 8****. Peptidoglycan content.**
   **A.** UPLC muropeptides separation. A representative muropeptide profile is presented for cells grown for the same amount of time in M9-MM supplemented (bottom panel) or not (top panel) with 0.2% L-Ara. Graphs were normalized by scaling the chromatograms to the maximum intensity measured in each run. **B.** Peptidoglycan quantification in non-treated and L-Ara treated cells.

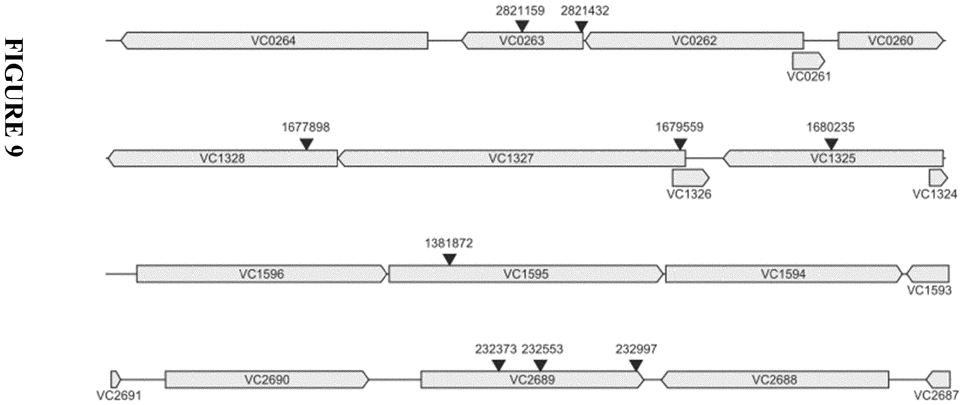

**Figure 9****. Maps of the regions of the chromosomes showing the location of Tn insertions in L-Ara insensitive mutants.** The arrows represent the sites of insertion.

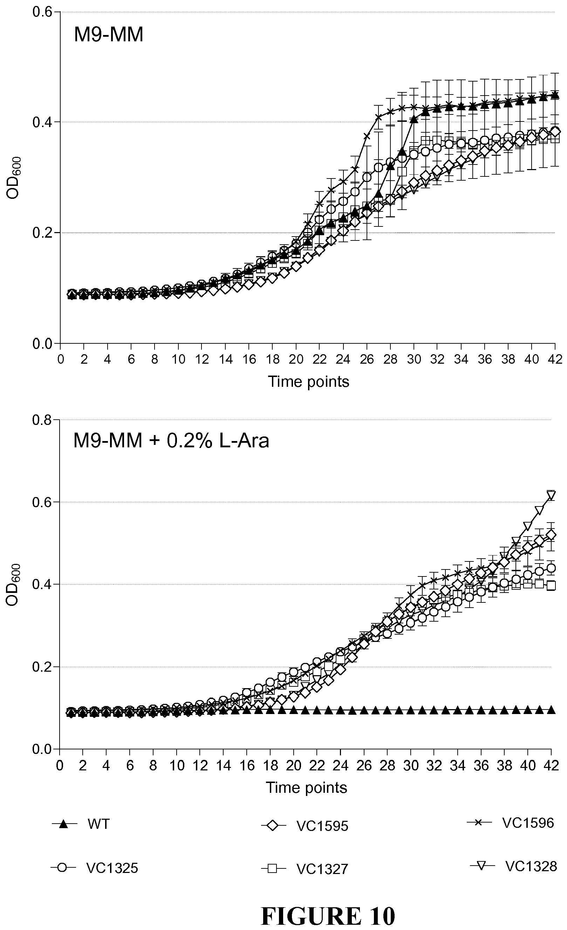

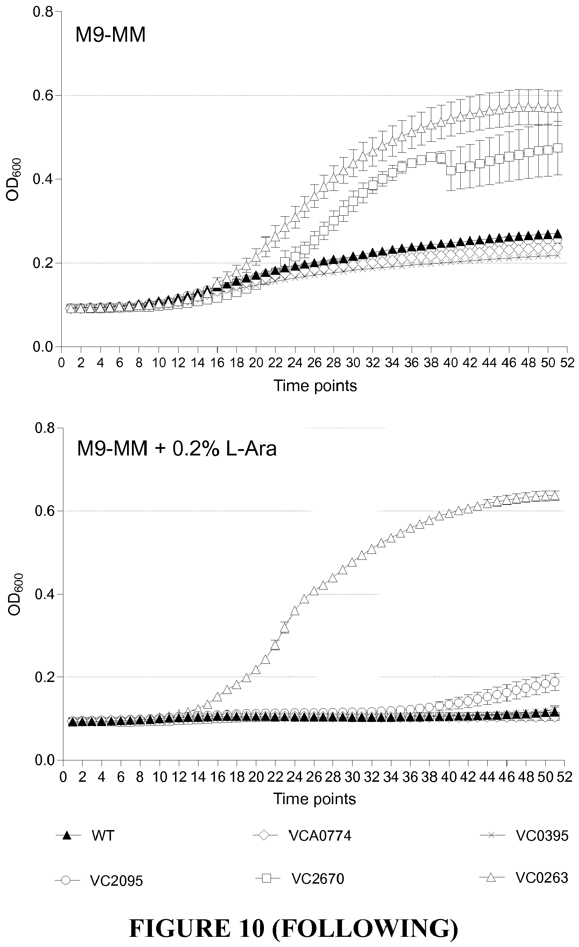

**Figure 10****. Genes involved in the production of toxic L-Ara by-products.**
   Growth profiles in M9-MM of *V. cholerae* strains carrying Tn inactivated mutants of genes belonging to the galactose Leloir and glycolysis pathways. The optical density at 600 nm over time is the mean of three independent replicates; the standard deviation is represented for each time point.

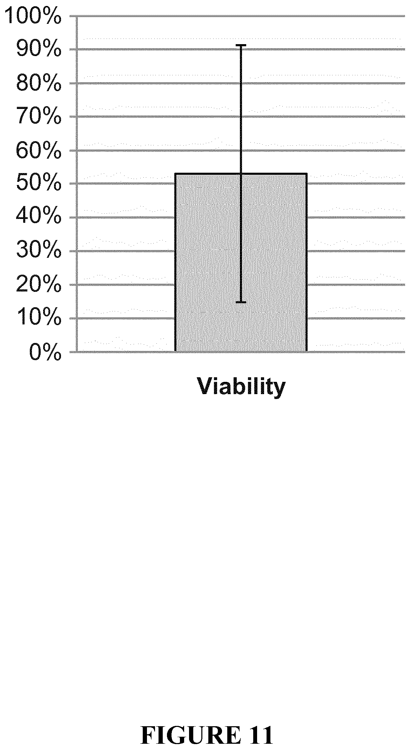

**Figure 11****. Viability of cells after exposure to L-Ara.**
   *V. cholerae* cells were grown in M9-MM for 2 hours (t0) and after addition of 0.2% L-Ara for other 5 hours (t1). CFU were counted at t0 and t1, the viability is given by the ratio CFU at t1 / CFU at t0.

### EXAMPLES

### MATERIALS & METHODS

### Plasmids and strains

Bacterial strains and plasmids used in this example are listed in Table 4. Strains were rendered competent by the insertion of *hapR* by specific transposition and constructed by natural transformation. Engineered strains were confirmed by PCR.

**Table 4 (List of strains and plasmids used in the example):**

| **Name** | **Relevant genotype or features** | **Reference** |
|---|---|---|
| EGV299 | MCH1 *ChapR ΔlacZ*::(P_{BAD}::*YGFP-slmA-lacZ-Sh ble*) zeo^{R}, gm^{R} | This example |
| EPV50 | N16961 *ChapR ΔlacZ* gm^{R} | [David *et al.*] |
| VC0263 | N16961 Tn inactivated *vc0263* | This example |
| VC0395 | C6706 Tn inactivated *vc0395* | [Cameron *et al.*] |
| VC1595 | C6706 Tn inactivated *vc1595* | [Cameron *et al.*] |
| VC1596 | C6706 Tn inactivated *vc1596* | [Cameron *et al.*] |
| VC2095 | C6706 Tn inactivated *vc2095* | [Cameron *et al.*] |
| VC2670 | C6706 Tn inactivated *vc2670* | [Cameron *et al.*] |
| VCA0774 | C6706 Tn inactivated *vca0774* | [Cameron *et al.*] |
| SM10 λ *pir* | *kan^{R}*, *this-1, thr, leu, tonA, lacY, supE, recA::RP4-2-Tc::Mu, pir* | [Simon *et al.*] |
| pEP348 | P_{BAD}::*YGFP*-*slmA*-*lacZ*-*Sh ble* flanked by the upstream and downstream regions of lacZ; ori pUC; zeo^{R} amp^{R} | This example |
| pSC189 | kan^{R}, amp^{R}, C9 transposase | [Chiang *et al.*] |

### Growth curves

Cells were grown at 30°C in M9 minimal medium supplemented with 0.2% fructose and 1 µg/ml thiamine (M9-MM) and M9-MM + 0.1% casamino acids (M9-MM + CAA) in a 96-well microtiter plate and the optical density at 600 nm followed over time in a Tecan plate reader. The growth curves plotted are the average of three replicates; the standard deviation is represented for each time point.

### Microscopy

Cells were grown to exponential phase and spread on a 1% (w/v) agar pad (ultrapure agarose, Invitrogen) for analysis. For snapshot analyses, cells images were acquired using a DM6000-B (Leica) microscope. For time-lapse analyses the agarose pad was made using M9-MM and images were acquired using an Evolve 512 EMCCD camera (Roper Scientific) attached to an Axio Observe spinning disk (Zeiss).

### Transposon mutagenesis screen

The transposon mutagenesis was performed conjugating the *E. coli* strain SM10 λ *pir*/pSC189, which carries a mini-Himar transposon associated to a kanamycin resistance, with the *V. cholerae* strain EGV299. Tn mutants were selected on M9-MM plates containing kanamycin, 0.1% L-Ara and 0.004% X-Gal (5-bromo-4-chloro-3-indolyl-β-D-galactopyranoside) incubated overnight at 30°C. Mutants able to grow as blue colonies on plate were isolated and inspected at the microscope for growth and morphology in presence of L-Ara. An arbitrary PCR followed by DNA sequencing was performed to determine the transposon insertion site.

### L-Ara survival assay

An EPV50 (derived from El Tor isolate N16961) overnight culture was diluted 200 times in M9-MM, followed by 2 hours of growth at 30°C before 0.2% L-Ara was added and growth continued for other 5 hours. Cells were checked for complete transition to spherical morphology at the microscope. Serial dilutions of t0 (before L-Ara addition) and t1 (after L-Ara treatment) samples were plated on Luria Bertani (LB) agarose plates and the number of colonies used to calculate the CFU at t0 and t1. The ratio CFU t1 / CFU t0 is used to calculate the percentage of cells able to survive L-Ara treatment and revert to proliferating cells.

### Muropeptide analysis

EPV50 overnight cultures were diluted 200 times in M9-MM and grown at 30°C. 100 ml were centrifuged after 7 hours of growth and 1L of culture to which 0.2% L-Ara was added after 2 hours was harvested after further 5 hours of incubation. Cells were checked for complete transition to spherical morphology at the microscope. Previously described methods were followed for muropeptide isolation and ultra-performance liquid chromatography (UPLC) analysis [Desmarais *et al.* and Möll *et al.*]*.* After boiling for 2 hours cell pellets with SDS (sodium dodecyl sulfate) the lysates were left stirring over night at room temperature. Cell wall material was pelleted, washed with MQ water to remove the SDS, and digested with pronase E to remove Braun's lipoprotein. Purified peptidoglycan was re-suspended in MQ water and treated overnight with muramidase at 37°C. Soluble muropeptides were reduced with sodium borohydride and the pH then adjusted to 3.5 with phosphoric acid. Samples were injected in an ultra-performance liquid chromatography (UPLC) system to obtain the muropeptide profiles. UPLC separation was performed on a Waters UPLC system equipped with an ACQUITY UPLC BEH C18 Column, 130 Å, 1.7 µm, 2.1 mm × 150 mm (Waters) and a dual wavelength absorbance detector using a linear gradient from buffer A (phosphate buffer 50 mM, pH 4.35) to buffer B (phosphate buffer 50 mM, pH 4.95, methanol 15% (vol/vol)) in a 22-min run with a 0.25 mL/min flow. Elution of muropeptides was detected at 204 nm. Identity of the peaks was assigned by comparison of the retention times and profiles to other chromatograms in which mass spectrometry data has been collected. The relative amounts of the muropeptides and the percentage of cross-linkage were calculated as described by Glauner *et al.* To calculate the amount of peptidoglycan per cell, the total area of the muropeptides was normalized to the OD of the culture. All values are the means of three independent experiments.

### RESULTS

### L-Arabinose is toxic in V. cholerae

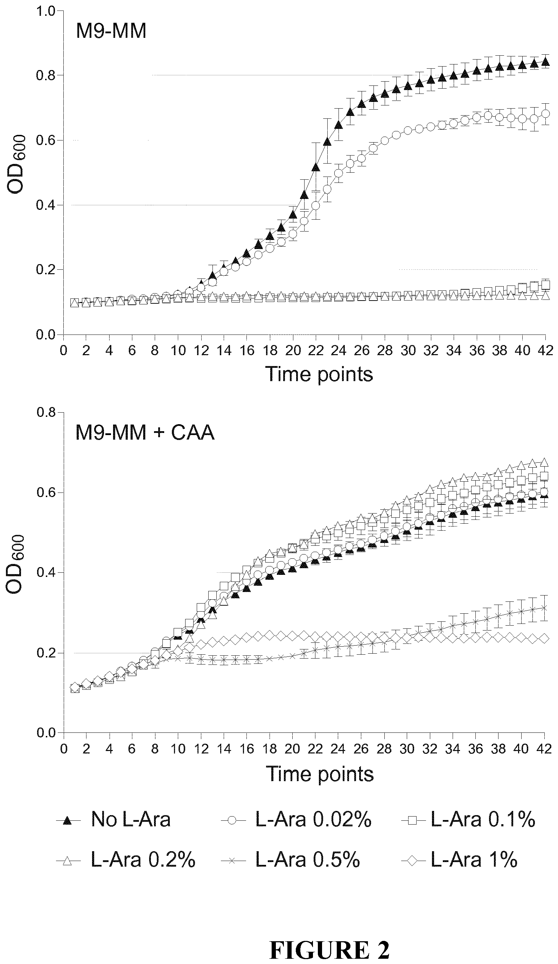

During experiments in which inventors used an arabinose-inducible promoter to express fluorescently tagged proteins in *V. cholerae*, they occasionally observed the presence of spherical cells. Furthermore, when working in mutant backgrounds with a reduced fitness (e.g. *V. cholerae* strain MCH1 expressing SlmA [Galli *et al.*]), the entire cell population consisted of spherical cells upon induction with a concentration of L-Ara as low as 0.01% (data not shown). This phenotype was not caused by the expression of the proteins but by the inducer L-Ara itself. Indeed, a wild type *V. cholerae* strain, which naturally does not carry *araC* and *araBAD*, responded similarly to the presence of L-Ara: the cell population of a wild type *V. cholerae* (El Tor isolate N16961) incubated for few hours with 0.1% L-Ara was composed by spheres with a heterogeneous diameter size (Figure 1A). To better understand this phenotype, inventors grew *V. cholerae* cells to exponential phase in different liquid media and added increasing concentrations of L-Ara. After a few hours, they inspected the cells morphology at the microscope. Cells grown in M9-minimal medium (M9-MM) appeared with a normal rod-shape in the absence or with 0.02% L-Ara (concentration that is usually used to express proteins from a P_{BAD} promoter) but became completely round in the presence of 0.1% of this inducer (Figure 1B). In cultures grown in M9-MM supplemented with casamino acids (CAA) spherical cells started to appear at 0.2% L-Ara and all cells exhibited the spherical phenotype at 0.5% L-Ara (Figure 1B). To see the effect of L-Ara on cell proliferation and growth, overnight cultures were diluted into fresh media containing increasing amounts of the inducer. The OD of these cultures was monitored over time (Figure 2). L-Ara arrested *V*. *cholerae* cell growth in M9-MM and M9-MM+CAA, reflecting the results obtained by cell morphology analysis. Taken together these results show that L-Ara affects *V. cholerae* morphology and has a detrimental effect on cell growth.

Other sugars or carbon sources were tested for similar effects on cell shape and growth, but none of them, including D-Arabinose, affected *V. cholerae* growth or morphology at the concentration tested (Table 1 and Figure 5).

**Table 1: Tested carbon sources**

| **Carbon source** | **Spherical cells** |
|---|---|
| D-Arabinose | - |
| L-Arabinose | + |
| L-Rhamnose | - |
| Gluocose | - |
| Galactose | - |
| Glycerol | - |
| Sucrose | - |

The only carbon source with deleterious effects on cell growth or cell morphology was L-Ara.

### L-Arabinose induces the formation of spherical cells

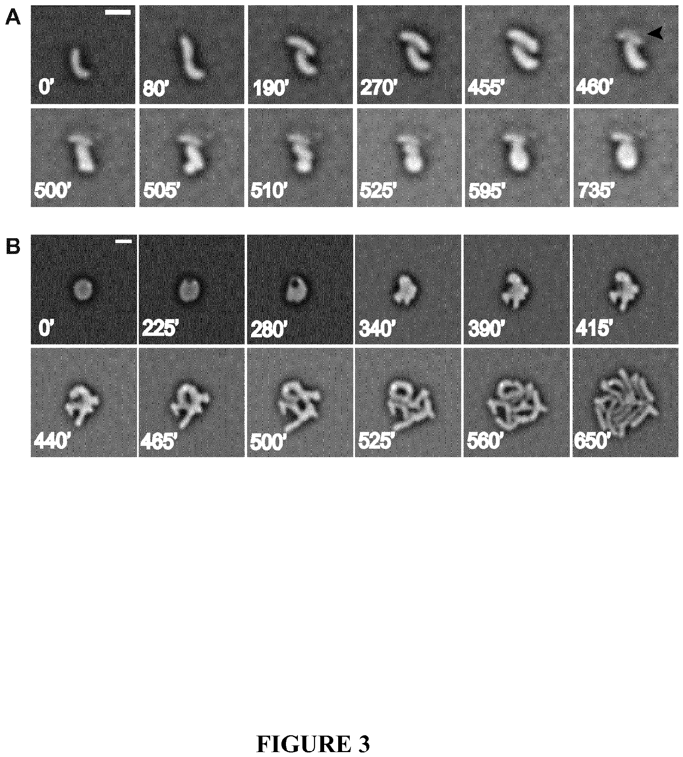

The spherical shape of L-Ara treated cells reminded that described for Cell Wall-Deficient (CWD)-forms that have lost completely (protoplast-type) or almost entirely (spheroplast-type) the peptidoglycan layer [Allan *et al.*]*.* However, contrary to *E. coli* cefsulodin- or lysozyme-induced L-forms [Joseleau-Petit *et al.*, Ranjit *et al.*, and Cambré *et al*.], *V. cholerae* spherical cells can survive without osmotic stabilizing media. Inventors performed time-lapse experiments to analyze the process of transition from rods to spheres (Figure 3A). Upon exposure to L-Ara, blebs were formed at the bacterial surface. Blebbing seemed to be distributed all along the cell, with no particular preference for mid-cell or the poles (Figure 6). As the blebs increased in size, the original cells were assimilated into the spheres, supposedly following cell wall degradation. Rod cells failing to transition to spheres lysed (Figure 3A, top cell marked with an arrow). A similar morphological phenotype, consisting of viable but non-dividing spherical cells, was recently reported for *V. cholerae* cells exposed to antibiotics inhibiting cell wall synthesis [Dörr *et al.*]*.* However, transition to spheres in cells treated with antibiotics targeting the cell wall occurred in the large majority from blebs located at mid-cell [Dörr *et al*.], suggesting that the enzymes preferentially involved in cleaving the cell wall to allow bleb formation might be different in the two pathways.

Reversion to rod shape after removal of L-Ara was followed performing time-lapse video microscopy (Figure 3B). Protrusions started to form from multiple positions around the sphere surface, elongating outward the sphere before being segregated into multiple branched and thickened cells. After few generations, the original rod shape was recovered, even though the time required for the initial formation of protruding walled cells could greatly differ between cells. In some cases, the recovery process started almost immediately after removal of L-Ara whereas in other cases it took few hours, but in all cases when the reversion process had begun it was completed in only a few cell division events. The variability in the time required to revert from non-dividing spheres to proliferating rod cells upon removal of L-Ara is reflected by the heterogeneous size distribution of colonies when a L-Ara treated sample is spread on plates without L-Ara (Figure 7, right panel). On the contrary, an untreated sample gives rise to colonies of similar sizes (Figure 7, left panel). The percentage of survival to L-Ara treatment is around 50% (Figure 11). Loss of viability is probably due both to cells unable to transition into spheres that lyse and to spherical cells unable to revert to rod.

### Peptidoglycan structure in L-Ara-treated spherical CWD-cells

Loss of rod-shape and formation of spherical cells suggest a process of cell wall degradation and remodeling. Inventors grew in parallel L-Ara treated and non-treated cells to determine the composition of the peptidoglycan present in *V. cholerae* spherical cells. Cells were collected at the time point at which they all look consistently spherical after being incubated with L-Ara. Peptidoglycan isolation followed by integration of the area of the obtained UPLC-profiles (Figure 8A) shows that the amount of peptidoglycan per cell in spherical L-Ara-treated bacteria is around 10 times lower than the amount present in rod-shaped cells (Figure 8B). In addition, quantification of the muropeptides (summarized in Table 2) shows that compared to non-treated cells, in spherical L-Ara-treated cells the amount of DAP-DAP cross-linked muropeptides increases significantly and glycan chains appear to be on average around 2 times shorter.

**Table 2: Muropeptides analyses**

| **Muropeptide group** | **Non-treated** | **L-Ara-treated** |
|---|---|---|
| Monomers (%) | 48.1 (±0.6) | 40.2 (±3.2) |
| Dimers (%) | 43.1 (±1.9) | 47.2 (±3.1) |
| Trimers (%) | 8.8 (±2.5) | 12.6 (±4.8) |
| Anhydro-muropeptides (%) | 14.8 (±2.3) | 21.9 (±3.7) |
| DAP-DAP cross-linked muropeptides (%) | 4.9 (±0.7) | 7.5 (±0.9) |

| **Peptidoglycan feature** | **Non-treated** | **L-Ara-treated** |
|---|---|---|
| Total cross-linkage (%) | 37.7 (±1.4) | 47.2 (±5.0) |
| Average glycan chain length | 11.7 (±1.4) | 7.5 (±1.1) |

Glycan chain length is calculated based on the number of anhydro-muropeptides [Glauner *et al.*]*.* Anhydro-muropeptides are the result of the activity of lytic transglycosylases [van Heijenoort *et al.*, and Vollmer *et al*.], reduction of average glycan chain length suggests an increase of their activity in the presence of L-arabinose. Likewise, the increase of DAP-DAP cross-linkage seems to indicate an increase in the activity of L,D-transpeptidases, which have been described to be specific for generating this unusual kind of cross-linkage [Magnet *et al.*]*.* The peptidoglycan structure of *V. cholerae* L-Ara-induced CWD-cells is remarkably similar to that of *E. coli* cefsulodin-induced L-forms [Joseleau-Petit *et al*.].

### A by-product of L-Arabinose triggers the morphological response

To identify the factors involved in the response to L-Ara and transition to spherical cells inventors performed a random transposon (Tn) mutagenesis screen. They screened for Tn mutants that could form colonies on M9-MM plates containing 0.1% L-Ara. As a receiver strain in the screening, they elected to choose a strain highly affected by L-Ara, fully transitioning to spheres even at low concentrations (0.01% L-Ara). They chose a MCH1 strain expressing from a P_{BAD} promoter a SlmA-YGFP fusion translationally in frame with LacZ. Increased levels of SlmA in MCH1 reduce the strain fitness [Galli *et al*.], rendering it very sensitive to L-Ara and hence greatly reducing the number of false positives. Preferably, they were looking for mutants that could still uptake L-Ara from the medium. For this reason, they took advantage of LacZ expression and selected colonies of blue colour on L-Ara X-Gal plates. Positive candidates were additionally inspected for resistance to L-Ara in liquid medium and cell morphology at the microscope. They constructed a Tn library of approximately 300,000 mutants and 9 clones were characterized as insensitive to L-Ara and concurrently expressing SlmA-YGFP and LacZ. They mapped the Tn insertion loci and identified 6 inactivated genes (Table 3 and Figure 9).

**Table 3: Tn-based suppressor screening results**

| **Gene** | **Putative function** | **N. hits** |
|---|---|---|
| *vc0263* | Galactosyl-transferase | 2 |
| *vc1325* | Galactoside ABC transporter, periplasmic D-galactose/D-glucose binding protein | 1 |
| *vc1327* | Galactoside ABC transporter, ATP-binding protein | 1 |
| *vc1328* | Galactoside ABC transporter, permease protein | 1 |
| *vc1595* | Galactokinase | 1 |
| *vc2689* | 6-Phosphofructokinase, isozyme I | 3 |

The suppressor capacity of the genes identified in the screening was confirmed using the corresponding knocked out mutant strains from a *V. cholerae* mapped Tn library [Cameron *et al.*] or, if the strain corresponding to the inactivated gene of interest was missing (*vc0263*), transferring the mapped Tn into a wild-type background. Gene *vc2689* could not be inactivated in a wild-type background, even though in the screening was recovered in three independent clones. Probably the gene is essential in *V. cholerae* and cells need an additional mutation in order to be able to survive without it. For all the other targets inactivation of the mapped genes restored growth and rod-shape morphology in the presence of L-Ara (Figure 10). The majority of targeted genes was involved in the uptake or catabolism of galactose. VC1325, VC1327 and VC1328 are putative homologues of *E. coli* MglB, MglA and MglC, respectively. The three genes form an operon encoding the components of the ABC galactose transport system: MglB is the periplasmic binding protein, MglA the ATP-binding protein and MglC the permease, integral membrane protein [Harayama *et al.*, and Hogg *et al.*]*.* This suggests that in *V. cholerae*, which does not carry enzymes specialized for arabinose import or degradation, L-Ara can enter the cell mainly through the galactose transporter. However, small L-Ara amounts must still be able to access the cell through a different transporter, especially in the presence of high extracellular arabinose levels. Indeed, SlmA-YGFP and LacZ were expressed in the screening, but likely the amount of L-Ara entering the cells was not high enough to induce a toxicity response. In *Sinorhizobium meliloti* the arabinose transporter AraABC has been described to play a role in galactose uptake [Geddes *et al*.], supporting a role for the galactose transporter in recognizing arabinose. The other suppressors are part of the galactose catabolic pathway and glycolysis. The suppressor distribution along the galactose Leloir and glycolysis pathways (Figure 4) suggests that arabinose can be processed until it forms a product that cannot be further metabolized. This by-product likely induces a toxic response and the formation of CWD-spherical cells. After entering the cell, L-Ara is recognized as a substrate by VC1595, putative homologue of *E. coli* galactokinase GalK, the first enzyme of the Leloir pathway of galactose metabolism [Kalckar *et al*.], then processed by the galactose 1-phosphate uridylyltransferase VC1596, putative homologue of *E. coli* GalT, and the phosphoglucomutase VC2095, putative homologue of *E. coli* Pgm, before entering the glycolysis pathway. The gene *vc1595* was recovered in the Tn suppressor screening whereas Tn inactivated *vc1596* and *vc2095* from a *V. cholerae* mapped Tn library [Cameron *et al.*] were identified as suppressors when tested for L-Ara sensitivity in cell morphology and growth experiments (Figure 10). Growth of a *vc2095* mutant was very slow in presence of L-Ara, even though rod-cell morphology was completely restored. The partial growth defect could explain why inventors did not recover it in the suppressor screening. Tn inactivation of *vc0395* and *vca0774*, putative homologues of *E. coli* GalU and GalE, respectively, did not suppress L-Ara induced growth inhibition (Figure 10). In the glycolysis metabolic pathway, VC2689, putative homologue of *E. coli* 6-phosphofructokinase PfkA, was identified as a suppressor in three independent clones. The proteins involved in the successive reactions were essential and could not be tested, with the exception of VC2670, putative homologue of *E. coli* triosephosphate isomerase TpiA, which could not suppress L-Ara growth inhibition (Figure 10). Likely, *V. cholerae* PfkA or one of the enzymes following it in glycolysis catalyzes the formation of a product that cannot be further metabolized, eliciting a stress response mechanism and the resultant formation of spheroplast-like cells. The remaining gene mapped in the L-Ara Tn screening, *vc0263*, encodes a putative homologue of *E. coli* WcaJ, the initiating enzyme for colanic acid synthesis, described also to act as a galactose-1-phosphate transferase *in vitro* [Patel *et al.*]*.*

### DISCUSSION

Inventors herein reveal that in *V. cholerae* L-Ara induces a toxic response that kills a substantial fraction of treated cells (Figure 11), by inhibiting cell proliferation and inducing formation of spheroplast-like cells. This phenotype is more readily revealed in poor growth conditions or in strains with a reduced fitness and it appears to be strictly dependent to the presence of L-Ara, since all other sugars or carbon sources tested did not generate any defect in cell growth or morphology. The spherical cells formed in the presence of L-Ara were not able to proliferate in liquid or on solid medium, but reverted to the parental shape once the sugar was removed. The effect of L-Ara is similar to the effects of cefsulodin, a β-lactam antibiotic that specifically inhibits PBP1A and PBP1B [Dörr *et al.*, Joseleau-Petit *et al.*, and Cambré *et al*.], and fosfomycin, an antibiotic that inhibits synthesis of the cell wall precursor lipid [Mercier *et al.*]*.* The analysis of the muropeptide composition in *V. cholerae* L-Ara induced spherical cells showed that they are still surrounded by a residual layer of peptidoglycan whose structure is very similar to that described for cefsulodin-treated *E. coli* [Joseleau-Petit *et al.*]*.* The increase in DAP-DAP cross-linkage suggests that *V. cholerae* L,D-transpeptidase activity (LdtA [Cava *et al*.]) is stimulated by the presence of L-Ara. The dramatic decrease in the chain-length average and corresponding increase in anhydro-muropeptides hint to a role of lytic transglycosylases in remodeling the peptidoglycan structure, cleaving the peptidoglycan in shorter chains and as a result releasing anhydro-forms. The increase in the activity of lytic transglycosylases, such as a lysozyme, can in addition cause lysis of cells not transitioning to spheres in the presence of L-Ara. Inventors believe that addition of glycoside hydrolases, such as lysozyme, to the formulations will improve the bactericidal and bacteriostatic effects of L-Ara.

The present example suggests that the toxicity on *V. cholerae* strains is linked to two particularities of the bacterium. First, *V. cholerae* lacks a *bona fide* L-Ara catabolic pathway. Second, the random transposon (Tn) mutagenesis study suggests that the *V. cholerae* galactose catabolic pathway wrongly imports and processes L-Ara into a toxic intermediate. The last glycolytic enzyme identified in the inventors' suppressor screening is the putative homologue of 6-phosphofructokinase. It is possible that after this catalytic reaction L-Ara is converted into a phosphorylated sugar intermediate that cannot be further metabolized, eliciting growth inhibition and formation of spheroplasts as a survival response.

The known absence of any side effects of L-Ara diets in humans, such as mycosis, for example candidiasis, or of intestinal disorders such as diarrhea, nausea or vomiting, makes it an advantageous prophylactic agent. Moreover, it considerably reduces the risk of development of bacterial resistance in bacteria distinct from the targeted bacterial strain.

### REFERENCES

Allan EJ, Hoischen C, Gumpert J. Bacterial L-forms. Adv Appl Microbiol. 2009;68: 1-39. doi:10.1016/S0065-2164(09)01201-5
Cambré A, Zimmermann M, Sauer U, Vivijs B, Cenens W, Michiels CW, et al. Metabolite profiling and peptidoglycan analysis of transient cell wall-deficient bacteria in a new Escherichia coli model system. Environ Microbiol. 2015;17: 1586-1599. doi:10.1111/1462-2920.12594
Cameron DE, Urbach JM, Mekalanos JJ. A defined transposon mutant library and its use in identifying motility genes in Vibrio cholerae. Proc Natl Acad Sci USA. 2008;105: 8736-8741. doi:10.1073/pnas.0803281105
Cava F, de Pedro MA, Lam H, Davis BM, Waldor MK. Distinct pathways for modification of the bacterial cell wall by non-canonical D-amino acids. EMBO J. 2011;30: 3442-3453. doi:10.1038/emboj.2011.246
Chiang SL, Runbin EJ. Construction of a mariner-based transposon for epitope-tagging and genomic targeting. Gene. 2002;296: 179-85
David A, Demarre G, Muresan L, Paly E, Barre F-X, Possoz C. The two Cis-acting sites, parS and oriC1, contribute to the longitudinal organization of Vibrio cholerae chromosome I. PLoS Genet. 2014;10: e1004448. doi:10.1371/journal.pgen.1004448
Desmarais SM, De Pedro MA, Cava F, Huang KC. Peptidoglycan at its peaks: how chromatographic analyses can reveal bacterial cell wall structure and assembly. Mol Microbiol. 2013;89: 1-13. doi:10.1111/mmi.12266.
Dörr T, Davis BM, Waldor MK. Endopeptidase-mediated beta lactam tolerance. PLoS Pathog. 2015;11: e1004850. doi:10.1371/journal.ppat.1004850
Galli E, Poidevin M, Le Bars R, Desfontaines J-M, Muresan L, Paly E, et al. Cell division licensing in the multi-chromosomal Vibrio cholerae bacterium. Nat Microbiol. 2016;1: 16094. Geddes BA, Oresnik IJ. Inability to catabolize galactose leads to increased ability to compete for nodule occupancy in Sinorhizobium meliloti. J Bacteriol. 2012;194: 5044-5053. doi:10.1128/JB.00982-12
Glauner B, Höltje JV, Schwarz U. The composition of the murein of Escherichia coli. J Biol Chem. 1988;263: 10088-10095.
Guzman LM, Belin D, Carson MJ, Beckwith J. Tight regulation, modulation, and high-level expression by vectors containing the arabinose PBAD promoter. J Bacteriol. 1995;177: 4121-30.
Harayama S, Bollinger J, Iino T, Hazelbauer GL. Characterization of the mgl operon of Escherichia coli by transposon mutagenesis and molecular cloning. J Bacteriol. 1983;153: 408-415.
Hogg RW, Voelker C, Von Carlowitz I. Nucleotide sequence and analysis of the mgl operon of Escherichia coli K12. Mol Gen Genet MGG. 1991;229: 453-459.
Joseleau-Petit D, Liébart J-C, Ayala JA, D'Ari R. Unstable Escherichia coli L forms revisited: growth requires peptidoglycan synthesis. J Bacteriol. 2007;189: 6512-6520. doi:10.1128/JB.00273-07
Kalckar HM, Kurahashi K, Jordan E. HEREDITARY DEFECTS IN GALACTOSE METABOLISM IN ESCHERICHIA COLI MUTANTS, I. DETERMINATION OF ENZYME ACTIVITIES. Proc Natl Acad Sci USA. 1959;45: 1776-1786.
Lee N, Francklyn C, Hamilton EP. Arabinose-induced binding of AraC protein to araI2 activates the araBAD operon promoter. Proc Natl Acad Sci USA. 1987;84: 8814-8818. Magnet S, Dubost L, Marie A, Arthur M, Gutmann L. Identification of the L,D-transpeptidases for peptidoglycan cross-linking in Escherichia coli. J Bacteriol. 2008;190: 4782-4785. doi:10.1128/JB.00025-08
Mercier R, Kawai Y, Errington J. General principles for the formation and proliferation of a wall-free (L-form) state in bacteria. eLife. 2014;3. doi:10.7554/eLife.04629
Miyada CG, Stoltzfus L, Wilcox G. Regulation of the araC gene of Escherichia coli: catabolite repression, autoregulation, and effect on araBAD expression. Proc Natl Acad Sci U S A. 1984;81: 4120-4124.
Möll A, Dörr T, Alvarez L, Davis BM, Cava F, Waldor MK. A D, D-carboxypeptidase is required for Vibrio cholerae halotolerance. Environ Microbiol. 2015;17: 527-540. doi: 10.1111/1462-2920.12779.
Patel KB, Toh E, Fernandez XB, Hanuszkiewicz A, Hardy GG, Brun YV, et al. Functional characterization of UDP-glucose:undecaprenyl-phosphate glucose-1-phosphate transferases of Escherichia coli and Caulobacter crescentus. J Bacteriol. 2012;194: 2646-2657. doi:10.1128/JB.06052-11
Ranjit DK, Young KD. The Rcs stress response and accessory envelope proteins are required for de novo generation of cell shape in Escherichia coli. J Bacteriol. 2013;195: 2452-2462. doi:10.1128/JB.00160-13
Simon R, Priefer U, Puhler A. A broad host range mobilization system for in vivo genetic engeneering: transposon mutagenesis in Gram negative bacteria. Nat Biotech. 1983;1: 784-791. doi:10.1038/nbt1183-784
van Heijenoort J. Peptidoglycan hydrolases of Escherichia coli. Microbiol Mol Biol Rev MMBR. 2011;75: 636-663. doi:10.1128/MMBR.00022-11
Visick KL, Quirke KP, McEwen SM. Arabinose induces pellicle formation by Vibrio fischeri. Appl Environ Microbiol. 2013;79: 2069-2080. doi:10.1128/AEM.03526-12
Vollmer W, Joris B, Charlier P, Foster S. Bacterial peptidoglycan (murein) hydrolases. FEMS Microbiol Rev. 2008;32: 259-286. doi:10.1111/j.1574-6976.2007.00099.x
Wientjes FB, Wolringh CL, Nanninga N. Amount of Peptidoglycan in cell walls of Gram-negative bacteria. J. of Bacteriology. 1991; 173(23): 7684-7691

## Claims

1. A bacteriostatic or bactericidal product comprising at least one active compound consisting in L-arabinose (L-Ara) or a derivative thereof.

2. The bacteriostatic or bactericidal product according to claim 1, wherein the product is a medicament, a disinfectant, an antiseptic or a preservative agent.

3. The bacteriostatic or bactericidal product according to claim 2, wherein the medicament is an antibiotic.

4. A composition comprising a product according to claim 1 in a bacteriostatic or bactericidal effective amount and a diluent, carrier or support, preferably together with a glycoside hydrolase.

5. The bacteriostatic or bactericidal product according to claim 1 or the composition according to claim 4 for use for preventing or treating a bacterial infection in a subject.

6. Bacteriostatic or bactericidal product according to anyone of claims 1 to 3, composition according to claim 4, or bacteriostatic or bactericidal product or composition for use according to claim 5, wherein the targeted bacterium is a bacterium which does not express L-Ara catabolic enzymes such as a *Vibrio* genus bacterium.

7. Bacteriostatic or bactericidal product, composition, bacteriostatic or bactericidal product or composition for use according to claim 6, wherein the targeted bacterium or bacteria is (are) selected from *Vibrio cholerae*, *V. parahaemolyticus, V. vulnificus, V. aestuarianus, V. alginolyticus*, *V. splendidus*, *V. Harveyi* and any combination thereof.

8. A process for the bacteriostatic or bactericidal *in vitro* or *ex vivo* treatment of a medium infected with, or at risk of being infected with, a bacterium or bacteria as described in claim 7, wherein the process comprises a step of contacting the infected medium with a bacteriostatic or bactericidal product according to claim 1 or with a composition according to claim 4.

9. A method for assessing *in vitro* or *ex vivo* the sensitivity of a bacterial strain to L-Ara or to a derivative thereof, wherein the method comprises a step of exposing the bacterial strain to L-Ara or to a derivative thereof, and a step of assessing the shape, peptidoglycan content and/or proliferation rate of the bacteria, thereby assessing the sensitivity of the bacterial strain to L-Ara or to a derivative thereof.

10. The method of claim 9, wherein the conversion of bacteria into spheroplasts or protoplasts or a peptidoglycan content of the bacteria below 20%, indicates the sensitivity of the bacteria to L-Ara or to a derivative thereof, and wherein the absence of any change in the bacteria's shape, or a peptidoglycan content of the bacteria above 20%, indicates the resistance of the bacterial strain to L-Ara or to a derivative thereof.

11. The method of claim 9, wherein a bacterial proliferation rate below 60% indicates the sensitivity of the bacterial strain to L-Ara or to a derivative thereof and a bacterial proliferation rate above 60% indicates the resistance of the bacterial strain to L-Ara or to a derivative thereof.

12. Kit comprising at least one bacteriostatic or bactericidal product according to claim 1 or a composition according to claim 4, preferably together with at least one glycoside hydrolase, and a product and/or tool adapted to the detection and/or measure of L-Ara or of a derivative thereof in a medium or in a biological sample of a subject.

13. Use of a kit according to claim 12 for the bacteriostatic or bactericidal treatment of a subject or medium.

14. Use of a kit according to claim 12 or 13, wherein the subject is a human being.

15. Use of a kit according to claim 12 or 13, wherein the medium is selected from sea water, fresh water, tap water, a culture cell water or medium and an aquatic animal culture water or medium.
